Europäisches Patentamt

European Patent Office

Office européen des brevets

(19)

(11) **EP 0 874 994 B1**

(12) **EUROPÄISCHE PATENTSCHRIFT**

(45) Veröffentlichungstag und Bekanntmachung des Hinweises auf die Patenterteilung:
**02.05.2002 Patentblatt 2002/18**

(21) Anmeldenummer: **96939883.3**

(22) Anmeldetag: **21.11.1996**

(51) Int Cl.[7]: **G01N 33/68**, G01N 33/58

(86) Internationale Anmeldenummer:
**PCT/EP96/05141**

(87) Internationale Veröffentlichungsnummer:
**WO 97/19355 (29.05.1997 Gazette 1997/23)**

(54) **BESTIMMUNG DER ENDSTÄNDIGEN SIALINSÄURERESTE DES HUMAN-TRANSFERRIN-MOLEKÜLS**

DETECTION OF THE END-POSITION SIALIC ACID GROUPS OF THE HUMAN TRANSFERRIN MOLECULE

DETERMINATION DES GROUPES TERMINAUX ACIDE SIALIQUE DE LA MOLECULE TRANSFERRINE HUMAINE

(84) Benannte Vertragsstaaten:
**AT BE CH DE DK ES FI FR GB GR IE IT LI LU MC NL PT SE**

(30) Priorität: **22.11.1995 DE 19543569**

(43) Veröffentlichungstag der Anmeldung:
**04.11.1998 Patentblatt 1998/45**

(73) Patentinhaber: **Lo, Atou**
**70565 Stuttgart (DE)**

(72) Erfinder: **Lo, Atou**
**70565 Stuttgart (DE)**

(74) Vertreter: **Huber, Bernhard, Dipl.-Chem. et al**
**Weickmann & Weickmann**
**Patentanwälte**
**Kopernikusstrasse 9**
**81679 München (DE)**

(56) Entgegenhaltungen:
**US-A- 4 626 355          US-A- 5 179 004**

- **ARCH. BIOCHEM. BIOPHYS. (1987), 254(1), 1-8 CODEN: ABBIA4;ISSN: 0003-9861, 1987, XP000646703 SHIBUYA, NAOTO ET AL: "Fractionation of sialylated oligosaccharides, glycopeptides, and glycoproteins on immobilized elderberry ( Sambucus nigra L.) bark lectin"**

- **CHEMICAL ABSTRACTS, vol. 122, no. 15, 10.April 1995 Columbus, Ohio, US; abstract no. 182553, MATSUMOTO, KOJIRO ET AL: "Antibody - lectin sandwich enzyme immunoassay for determination of altered asparagine -linked sugar chains in serum transferrin of patients with hepatoma" XP002028961 & RINSHO KAGAKU (NIPPON RINSHO KAGAKKAI) (1994), 23(4), 292-8 CODEN: RIKAAN;ISSN: 0370-5633, 1994,**

- **ANAL. BIOCHEM. (1987), 165(2), 320-6 CODEN: ANBCA2;ISSN: 0003-2697, 1987, XP000646761 PEKELHARING, J. M. ET AL: "Lectin -enzyme immunoassay of transferrin sialovariants using immobilized antitransferrin and enzyme-labeled galactose-binding lectin from Ricinus communis"**

- **PROTIDES BIOL. FLUIDS (1984), VOLUME DATE 1983, 31, 115-18 CODEN: PBFPA6;ISSN: 0079-7065, 1984, XP000646760 PEKELHARING, J. M.: "An ELISA technique for measurement of transferrin glyco-variants in body fluids using lectins bound to a solid phase"**

- **UPSALA J. MED. SCI. (1981), 86(1), 39-53 CODEN: UJMSAP;ISSN: 0300-9734, 1981, XP000646787 CERVEN, CERIK ET AL: "Determination of terminal sugars in transferrin by radio- lectin immunoassay (RLIA)-a new microanalytical procedure"**

**Beschreibung**

Die vorliegende Erfindung betrifft die Bestimmung der endständigen Sialinsäurereste von humanem Transferrin. In einer Ausführungsform betrifft sie die Quantifizierung der endständigen Sialinsäure-Reste des Transferrin-Moleküls in Proben menschlicher Körperflüssigkeiten und insbesondere eine Interpretation von Veränderungen der chemischen Struktur des Transferrins, wie beispielsweise durch regelmäßigen und mißbräuchlichen Alkoholkonsum verursacht.

Zur Diagnose von Alkoholmißbrauch bzw. Alkoholabhängigkeit sind in der Medizin eine Reihe von Indikatoren bekannt. Die sogenannten "State-Marker" erlauben eine Abschätzung bezüglich der Alkoholaufnahme, wobei in Abhängigkeit des bestimmten Markers die Assoziation über einen kurzen Zeitraum (z.B. Blutalkoholspiegel) oder längerfristig (v.a. diverse Leberenzyme) besteht. Eine ausführliche Übersicht über Alkoholismus und geeignete Marker findet sich in M. Soyka (Herausgeber, Biologische Alkoholismusmarker, Chapman and Hall (1995)).

Als ein geeigneter Marker mit relativ hoher Spezifität hat sich eine abnorme Transferrinvariante herausgestellt. Die bisher veröffentlichten wissenschaftlichen Arbeiten haben gezeigt, daß diese Molekülveränderung in Form von fehlendem oder reduziertem Gehalt an Sialinsäureresten des Transferrin-Moleküls auftritt.

Das humane Serum-Transferrin ist ein Glykoprotein mit einem relativen Molekulargewicht von ca. 77.000 g/Mol und besteht aus einer einzelnen Peptidkette von 679 Aminosäuren, an die zwei Oligosaccharidketten angeheftet sind, die aus N-Acetylglucosamin, Mannose und Galaktose bestehen. Diese Kohlehydratketten besitzen zwei bis drei Antennen, an derem letzten Glied (Galactose) jeweils ein Sialinsäurerest gebunden ist.

Regelmäßiger Alkoholmißbrauch beeinträchtigt den Mechanismus der Übertragung der Sialinsäuren auf das Transferrin und es entstehen in Folge vermehrt Isoformen des Transferrins mit weniger als den normalerweise vier bis sechs endständig gebundenen Sialinsäureresten (desialinisiertes Transferrin, Sialinsäure-defizientes Transferrin, sialic deficient transferrin, SDT).

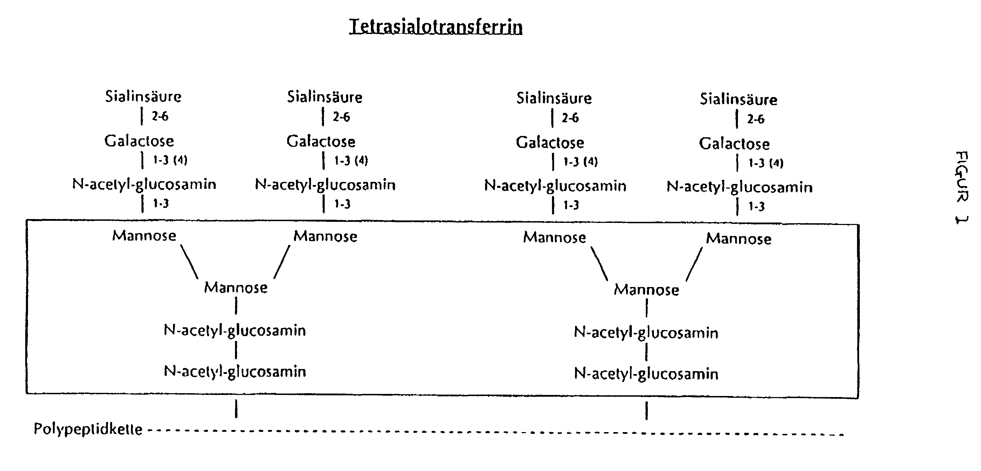

Gemäß De Jong et al. (1980) setzt sich die häufigste isoforme Struktur des Tetrasialotransferrins (Fig. 1) wie folgt zusammen:

4 endständige N-Acetyl-Sialinsäure-Reste, 4 Galactose-Reste, 8 N-Acetyl-Glucosamin-Reste und 6 Mannose-Reste in beiden Oligosaccharid-Seitenketten des Transferrinmoleküls.

Aus dem Stand der Technik bekannte Verfahren beruhen im wesentlichen auf der Auftrennung der unterschiedlich glykosylierten Transferrinderivate aufgrund ihrer Ladung, wie beispielsweise durch isoelectric focusing oder chromatographische Verfahren. Von den bekannten Methoden sind anzuführen:

1. Die 'Isoelectric Focusing'-Methode
Elektrophoretische Trennung der Isotransferrine entsprechend ihrem isoelektrischen Punkt (PI 6,1 - 5,1). (Helena Stibler et Stefan Borg, Pharmacol. Biochem. Behav. 1980, 13, Suppl. 1, 47-51).

2. Die chromatografische Methode mittels Ionenaustauscher
(Helena Stibler et al., Clinical and Experimental Research Sept./Oct. 1986, Vol. 10. No. S, 535-543).

3. Die HPLC-Methode,
Sättigung der Transferrine mit einem Eisensalz und anschließender Extraktion auf Kolonne, dann erfolgt eine densitometrische Bestimmung. (Strahler et al., J. of chromatographie 266,1983, 281-289).

4. Isocratische HPLC-Methode
Beruht auf der Trennung der Isotransferrine mittels kationischer Puffer.
(Joustra Marius et al., Patent Nr: EP 0 172 217 B1),(HPLC-Verfahren nach Jan Jeppson et al., Clin.Chemistry 39/IO; 2115-2120 (1993)

5. Turbidimetrische Methode der Firma AXIS
Basiert ebenfalls auf der Sättigung des Isotransferrins mittels Eisensalz oder einer heterogenen Immunoanalyse mit Trennung auf Kolonne gefolgt von einer turbidimetrischen Messung.
(Patent: WO 99119983 A 911226)

6. Die immunoenzymatische EIA-Methode durch eine Konjugation von Pharmacia
Verwendet einen monoklonalen Antikörper nach Sättigung des Isotransferrins mittels Eisensalz und Säulentrennung.
(O. Märtenson et al.).

7. Die RIA-Methode von Pharmacia:

EP 0 874 994 B1

Chromatografisch mittels Ionenaustauscher; nach Sättigung des Isotransferrins durch ein Eisensalz und quantitativ bestimmt durch Radio-Immuno-Analyse.

Die genannten Verfahren sind jedoch für den Anwender noch nicht zufriedenstellend. Problematisch ist insbesondere das Auftreten von Fehldiagnosen sowie eine sehr aufwendige Versuchsdurchführung.

Weiterhin ist aus dem Stand der Technik eine als Lectine bezeichnete Klasse von Proteinen oder Glykoproteinen bekannt, die an bestimmte Kohlehydratkonfigurationen bzw. an Glykoproteine, die solche Kohlehydratkonfigurationen tragen, binden können. Derartige Lectine wurden auf Basis ihrer Spezifität für bestimmte Kohlehydrate, beispielsweise zur blutgruppenspezifischen Agglutinierung von Erythrozyten eingesetzt.

Aus Uppsala, J. med. SCI; Bd. 86 (1), 1981, S. 39-53 ist ein Verfahren zur Bestimmung der endständigen Zuckerreste von Transferrin bekannt. Danach wird Transferrin an immobilisierte Anti-Transferrin-Antikörper gebunden und mit markierten Lectinen, die mit bestimmten endständigen Zuckerresten bindungsfähig sind, darunter das Limulus polyfemus Lectin, welches mit Sialinsäureresten reagiert, inkubiert. Jedoch wird ausdrücklich angegeben, dass dieses Lectin auch an Asialotransferrin-Anti-Transferrin-Komplex bindet, was eine Differenzierung zwischen Seren mit unterschiedlichen Mengen an pathologischen, Sialinsäure-armen Komponenten nicht zulässt.

Aus Arch. Bioche. Biophys, Bd. 254, 1987, Seiten 1-8 ist das Sambucus nigra Lectin und dessen Verwendung bei säulenchromatographischen Trennungsverfahren bekannt. Eine spezifische Bindefähigkeit mit end ständigen Sialinsäureresten von Human-Transferrin lässt sich für das Sambucus nigra Lectin daraus jedoch nicht entnehmen, ebensowenig eine Eignung zwischen Tetrasialotransferrin und Sialinsäure-defizienten Transferrinen zu unterscheiden.

Die der vorliegenden Erfindung zugrundeliegende Aufgabe bestand darin, ein Verfahren bereitzustellen, das die rasche und einfache Bestimmung endständiger Sialinsäurereste von Human-Transferrin gestattet.

Diese Aufgabe wird erfindungsgemäß gelöst durch ein Verfahren zur Bestimmung endständiger Sialinsäurereste von Human-Transferrin in einer Probenflüssigkeit nach einem Sandwich-Prinzip, welches dadurch gekennzeichnet ist, daß man die Probenflüssigkeit mit einem ersten, spezifisch mit Human-Transferrin bindefähigen Rezeptor inkubiert, den gebildeten Komplex von der Probenflüssigkeit abtrennt, mit einem zweiten, spezifisch mit endständigen Sialinsäureresten von Human-Transferrin bindefähigen Rezeptor nämlich Sambucus nigra-Lectin inkubiert, wobei der zweite Rezeptor an eine Markierung gebunden oder damit bindefähig ist, und den Komplex aus erstem Rezeptor, Human-Transferrin und zweitem Rezeptor über die Markierung bestimmt.

Im allgemeinen ist es aufgrund der bekannten und bewährten Technologie bevorzugt als ersten Rezeptor einen anti-Transferrin-Antikörper einzusetzen. Bevorzugt wird als erster Rezeptor ein polyklonaler anti-Transferrin-Antikörper eingesetzt. Derartige Antikörper sind kommerziell verfügbar (z.B. Sigma, No. T2027). Weiterhin sind jedoch andere mit Human-Transferrin spezifische Rezeptoren geeignet, soweit durch diese die Bindung von zweitem Rezeptor an die Sialinsäurereste im wesentlichen nicht beeinträchtigt wird. Beispielsweise ist die Verwendung von humanem Transferrin-Rezeptor denkbar.

Der zweite Rezeptor ist

- Sambucus nigra-Lectin, dessen Herstellung von Brökert et al. (Biochem. J. 221, 103-109 (1984)) beschrieben wurde.

In einer Ausführungsform des erfindungsgemäßen Verfahrens ist der erste Rezeptor an eine Festphase gebunden, wodurch das Transferrin bei der Inkubation an der Festphase fixiert wird. In einer anderen Ausführungsform kann der erste Rezeptor jedoch auch in Lösung vorliegen, aber über ein spezifisches Bindepaar, dessen einer Partner an die Festphase gebunden ist und dessen zweiter Partner an den Rezeptor gebunden ist, mit einer Festphase bindefähig sein. Geeignete Bindepaare sind dem Fachmann bekannt.

Als Festphase wird zweckmäßig eine Wand eines Reaktionsgefäßes, wie etwa ein Probenröhrchen, eine Mikrotiterplatte oder eine Küvette, verwendet. Weiterhin kann die Festphase aus teilchenförmigem Material, wie etwa Polystyrol- oder Magnetbeads, bestehen.

Ebenso kann der zweite Rezeptor direkt an die Markierung gebunden sein oder mittels eines spezifischen Bindungspaares mit der Markierung bindefähig sein. In einer bevorzugten Ausführungsform ist das spezifische Bindungspaar Biotin und Streptavidin bzw. Avidin, wobei dann typischerweise der zweite Rezeptor biotinyliert ist und die Markierung Streptavidin/Avidin trägt.

Die Inkubationen mit erstem Rezeptor und zweitem Rezeptor werden jeweils während eines Zeitraums von 10 bis 60 min, bevorzugt von 20 bis 40 min und bei einer Temperatur von 10 bis 40°C durchgeführt. Eine 30-minütige Inkubation bei Raumtemperatur liefert gute Ergebnisse.

Als Markierung für den zweiten Rezeptor sind im wesentlichen alle Nachweisverfahren geeignet, die in der Technik üblicherweise im Zusammenhang mit Immunassays verwendet werden.

Derartige Verfahren sind dem Fachmann bekannt und brauchen daher nicht ausführlich erläutert zu werden. Sie umfassen insbesondere enzymatische Verfahren, d.h. Verfahren, bei denen die Markierung ein Enzym ist und der Nachweis über die Bestimmung eines Substrats erfolgt. Übliche Enzyme, die für einen derartigen Zweck verwendet werden, umfassen z.B. Phosphatasen, wie etwa alkalische Phosphatase, Oxidasen, Peroxidasen, Dehydrogenasen wie etwa Glucose-6-Dehydrogenase, Hydrolasen, wie etwa Urease etc. Die enzymatische Reaktion erzeugt typischerweise ein chromogenes, lumineszierendes oder fluoreszierendes Substrat, das dann geeignet zumeist über photometrische Verfahren bestimmt werden kann. Geeignete Chromogene umfassen beispielsweise ABTS (2,2'-Azino-bis-(3-ethyl)-benzthiazolin-6-sulfonsäure), Orthophenyldiamin, Tetramethylbenzydin, 5-Amino-Salicylsäure etc. Enzymatische Verfahren umfassen auch z.B. eine durch die Substratreaktion verursachte Veränderung eines chemischen oder physikalischen Parameters, wie etwa der Lichtabsorption. Die Sialinsäure-Reste können somit auch durch UV-Methoden mit Alkohol-Dehydrogenase, Glucose-6-Dehydrogenase etc. und mittels den Coenzymen NAD, NADP etc. bestimmt werden oder mittels Verwendung einer Diaphorase in Anwesenheit von NADP und dem Chromogen Jodo-Nitrotetrazoliumviolett, um ein farbiges Formazan-Derivat zu bilden.

Neben einer Enzymmarkierung sind selbstverständlich andere Markierungsarten, wie etwa eine Radio-, Fluoreszenz- oder Lumineszenzmarkierung möglich; eine radioaktive Markierung ist jedoch aus Praktibilitätsgründen nicht bevorzugt. Die Durchführung eines Immunofluoreszenztestes ist beispielsweise bei Verwendung eines Konjugats von zweitem Rezeptor mit IFTC (Fluoreszin-isothiocyanat) möglich.

Als Probenflüssigkeit, die das zu bestimmende Transferrin enthält, wird üblicherweise eine Körperflüssigkeit, wie etwa Blut, Serum, Urin, Liquor, Glaskörperflüssigkeit, Galle, Aszites-Flüssigkeit etc. verwendet werden. Gegebenenfalls wird eine durch chemische oder physikalische Behandlung modifizierte Körperflüssigkeit verwendet, die jedoch bevorzugt frei von Antikoagulantien ist. Bevorzugt ist die Körperflüssigkeit Vollblut oder Serum.

Ein weiterer Gegenstand der Erfindung ist ein Verfahren zur Bestimmung von Sialinsäure-defizientem Transferrin in Körperflüssigkeiten, welches dadurch gekennzeichnet ist, daß man

(a) an einer Probe einer Körperflüssigkeit eine Bestimmung nach einem Verfahren eines der vorhergehenden Ansprüche durchführt,

(b) eine Bestimmung von endständigen Sialinsäureresten an mindestens einem Standard durchführt, der eine Substanz, umfassend mit dem zweitem Rezeptor bindefähige Sialinsäurereste in definierter Menge enthält, und

(c) den Gehalt von Sialinsäure-defizientem Transferrin auf Basis der Meßgrößen von Schritt (a) und Schritt (b) ermittelt.

Bevorzugt wird Schritt (b), d.h. die Bestimmung der Meßgröße für einen Standard mehrmals durchgeführt, wobei die Standards jeweils eine unterschiedliche Menge von mit dem zweitem Rezeptor bindefähigen Sialinsäureresten enthalten. Besonders bevorzugt werden drei bis sieben, am meisten bevorzugt fünf bis sechs Messungen mit unterschiedlichen Standards durchgeführt und aus den erhaltenen Ergebnissen eine Eichkurve ermittelt. Durch den Vergleich des gemessenen Wertes für die Probenflüssigkeit mit der Eichkurve läßt sich der Gehalt des Sialinsäure-defizienten Transferrins mit guter Genauigkeit bestimmen.

Wie für den Fachmann offensichtlich ist, werden bevorzugt geeignete Negativ- oder/und Positivkontrollproben parallel mitbestimmt.

Gemäß einer Ausführungsform verwendet man einen Standard, der Transferrin mit einer definierten Menge von Sialinsäureresten enthält. Darunter werden Körperflüssigkeiten, beispielsweise gepoolte Körperflüssigkeiten sowie geeignet formulierte Zusammensetzungen verstanden. Geeignete Konzentrationen der Sialinsäurereste in den Standards für die Erstellung der Eichkurve liegen in einem Bereich von 0 bis 50 μmol/dl, bevorzugt von 2,5 bis 25 μmol/dl.

Gemäß einer anderen Ausführungsform enthält der Standard eine von Transferrin verschiedene Substanz mit einer definierten Menge von mit dem zweiten Rezeptor bindefähigen Sialinsäureresten. Ein Beispiel für eine derartige Substanz ist ein festphasenseitiges Mucin oder/und ein festphasenseitiges Oligosaccharid mit definiertem Gehalt von mit dem zweiten Rezeptor bindefähigen Sialinsäureresten, wie z.B. ein von Sigma erhältliches Mucin (Sigma No. M3895, Mucin von Bovine Submaxillary Glands). Der Begriff "festphasenseitig" wird in dieser Beschreibung in der Bedeutung an eine Festphase gebunden (immobilisiert) oder damit bindefähig (immobilisierbar) verwendet.

Ein weiteres Beispiel ist N-Acetylneuramin-Lacto-N-neo-Tetraose c (α Neu 5 AC-(2-6)-βGal-(1-4)-βGlcNAc-(1-3)-βGal-(1-4)-Glc (Sigma Nr. A4814) in Kombination mit einer mit Concavalin A (Sigma Nr. C7275) beschichteten Festphase (spezifisch für α-D-Glycosyl-Bindungen).

Wenn als Standard eine Transferrin-enthaltende Flüssigkeit verwendet wird, erfolgt die Fixierung von Transferrin an der Festphase mittels erstem Rezeptor. Bei Verwendung eines Standards, der eine von Transferrin verschiedene Substanz enthält, ist wie für den Fachmann offensichtlich die Fixierung der Markierung über einen Komplex aus erstem Rezeptor und Transferrin nicht möglich. In diesem Fall ist daher die Sialinsäurereste-enthaltende Substanz festphasenseitig, d.h an eine Festphase gebunden oder damit bindefähig.

Das erfindungsgemäße Verfahren ist anwendbar zur Bestimmung von SDT in Proben von Körperflüssigkeiten von männlichen und weiblichen Spendern. Für gesunde Personen wurde in der Literatur ein physiologischer Transferringehalt von 260 mg/dl ± 15% berichtet. Überraschenderweise ergibt das erfindungsgemäße Verfahren auch für Schwangere korrekte Resultate.

Gemäß einem weiteren Aspekt betrifft die Erfindung auch die Verwendung von Sambucus nigra-Lectin in einem Verfahren zur Bestimmung von Sialinsäure-defizientem Transferrin.

Da wie bereits eingangs erwähnt das Sialinsäure-defiziente Transferrin ein zuverlässiger Alkoholismusmarker ist, kann das erfindungsgemäße Verfahren die Diagnostik im Zusammenhang mit Alkoholmißbrauch erheblich erleichtern.

Ein nochmals weiterer Gegenstand der vorliegenden Erfindung ist somit ein Diagnoseverfahren, bzw. die Verwendung des vorstehend genannten Verfahrens zur Diagnose von Alkoholmißbrauch oder Alkoholabhängigkeit. Zur Diagnose wird nach Bestimmung von Sialinsäure-defizientem Transferrin in einer Probe einer Körperflüssigkeit durch Vergleich mit Normalwerten eine pathologische Veränderung des Gehalts der endständigen Sialinsäurereste ermittelt. Dieser Vergleich erfolgt auf herkömmliche Weise, beispielsweise durch Mitführen von Vergleichsproben von Patienten und/oder Gesunden. Alternativ oder zusätzlich kann rechnerisch ermittelt werden, ob der erhaltene Wert innerhalb des Normalbereichs liegt.

Weitere Verwendungsmöglichkeiten in diesem Zusammenhang sind beispielsweise die Früherkennung von regelmäßigem Alkoholabusus und Alkoholismus, die Therapieüberwachung von Entzugspatienten, ein differentialdiagnostischer Einsatz zur Unterscheidung von alkoholinduzierten Erkrankungen (z.B. Leber) von nichtalkoholischen Schäden, eine labordiagnostische Entscheidungshilfe zur Einleitung einer Alkoholentzugs-Pharmakoprophylaxe in der Intensivmedizin, sowie allgemein in der Rechts- und Arbeitsmedizin, z.B. für gutachterliche Fragestellungen.

Gemäß einem nochmals weiteren Aspekt betrifft die Erfindung auch ein Reagenzienkit, das zur Bestimmung endständiger Sialinsäurereste von Transferrin geeignet ist. Ein derartiges Kit umfaßt in räumlich getrennter Anordnung festphasenseitigen, mit Human-Transferrin spezifisch bindefähigen ersten Rezeptor und markierten oder mit einer Markierung bindefähigen, spezifisch mit endständigen Sialinsäureresten von Human-Transferrin bindefähigen zweiten Rezeptor, nämlich Sambucus nigra-Lectin sowie gegebenenfalls eine mit dem ersten Rezeptor spezifische bindefähige Festphase, eine mit dem zweiten Rezeptor spezifisch bindefähige Markierung, ein Substrat für die Markierung sowie übliche Hilfs-, Puffer- und Zusatzstoffe.

Der erste Rezeptor ist bevorzugt ein anti-Transferrin-Antikörper, insbesondere ein polyklonaler anti-Transferrin-Antikörper.

Gegebenenfalls enthält das Reagenzienkit weiterhin mindestens einen Standard, der eine Substanz, umfassend mit dem zweiten Rezeptor bindefähige Sialinsäurereste in definierter Menge enthält, wie vorstehend beschrieben. Bevorzugt ist diese Substanz N-Acetylneuramin-Lacto-N-neo-Tetraose c ($\alpha$ Neu 5 AC-(2-6)-$\beta$Gal-(1-4)-$\beta$GlcNAc-(1-3)-$\beta$Gal-(1-4)-Glc (Sigma Nr. A4814), welches mit einer Concavalin A (Sigma Nr. C7275) beschichteten Festphase bindefähig ist, oder ein Mucin mit definiertem Gehalt von $\alpha$-anomerischen (2-6) Sialinsäureresten (z.B. Sigma No. M3895, Mucin von Bovine Submaxillary Glands).

Ein nochmals weiterer Gegenstand der vorliegenden Erfindung ist ein Teststreifen zur qualitativen und semiquantitativen Bestimmung von SDT, umfassend

(a) eine Probenauftragszone,
(b) mindestens eine Reaktionszone, enthaltend einen an einer Festphase immobilisierbaren ersten Rezeptor, der mit Human-Transferrin spezifisch bindefähig ist, und einen markierten zweiten Rezeptor, der mit endständigen Sialinsäureresten von Human-Transferrin spezifisch bindefähig ist nämlich Sambucus nigra-Lectin, und
(c) eine Nachweiszone zur Immobilisierung von erstem Rezeptor, enthaltend eine mit erstem Rezeptor spezifisch bindefähige Substanz.

Derartige Teststreifen sind dem Fachmann bekannt und bedürfen daher keiner ausführlichen Erläuterung. In kurzen Worten besteht ein derartiger Teststreifen aus einem Material, durch welches eine Probenflüssigkeit, enthaltend eine zu bestimmende Substanz z.B durch Kapillarkraft oder Schwerkraft migrieren kann, und welches bezüglich der zu bestimmenden Substanz 'inert ist. Nach Auftrag der Flüssigkeit in der Probenauftragszone migriert diese durch eine Reaktionszone, wobei ternäre Komplexe aus dem erstem Rezeptor, Transferrin und dem zweitem, markiertem Rezeptor gebildet werden, die danach in der Nachweiszone immobilisiert werden. Nicht gebundene Reaktanten und Substanzen migrieren über die Nachweiszone hinweg und werden in einer Endzone aufgefangen.

Der erste Rezeptor ist bevorzugt ein anti-Transferrin-Antikörper, insbesondere ein polyklonaler anti-Transferrin-Antikörper. Die Immobilisierung des ersten Rezeptors erfolgt bevorzugt über Biotin und Streptavidin/Avidin.

Zur Verwendung in derartigen Teststreifen geeignete Markierungen für den zweiten Rezeptor sind dem Fachmann bekannt und umfassen beispielsweise Goldmarkierung, Silbermarkierung oder Chromogene.

In einer bevorzugten Ausführungsform umfaßt der Teststreifen weiterhin mindestens eine Kontrollzone zur Negativkontrolle oder/und Positivkontrolle. Eine Negativkontrollzone entspricht im wesentlichen der Nachweiszone mit dem Unterschied, daß kein Bindungspartner zur Immobilisierung des ersten Rezeptors vorhanden ist. Eine Positivkontrollzone enthält zweckmäßig eine definierte Menge von mit dem zweitem Rezeptor bindefähigen, immobilisierten Sialinsäureresten. Für eine semiquantitative Bestimmung können mehrere Positivkontrollzonen mit unterschiedlichen Mengen von Sialinsäureresten vorhanden sein. Weitere Ausführungsformen und Layouts für den Teststreifen sind aus dem Stand der Technik bekannt.

Das erfindungsgemäße Verfahren kann darüber hinaus zur Bestimmung endständiger Sialinsäure-Reste sämtlicher natürlicher Proteine verwendet werden, die endständige Sialinsäure-Reste in 2-6-Stellung gebunden an β-Galactose und N-Acetyl-glucosamin enthalten.

Das erfindungsgemäße Verfahren wird durch die nachfolgenden Beispiele zusammen mit den beigefügten Figuren näher erläutert, in denen:

Figur 1 die Glykosylierung von Tetrasialotransferrin zeigt,

Figur 2 eine Standardkurve zur Auswertung einer Messung nach dem erfindungsgemäßen Verfahren zeigt.

In Figur 1 ist schematisch die Glykosylierung der häufigsten isoformen Struktur des Tetrasialotransferrins gezeigt. Die endständigen Kohlehydratreste sind Sialinsäurereste bzw. Acetylsialinsäurereste, die in $\alpha$2-6-Verknüpfung an Galactose gebunden sind, wobei die Galactose wiederum an N-Acetylglucosamin gebunden ist.

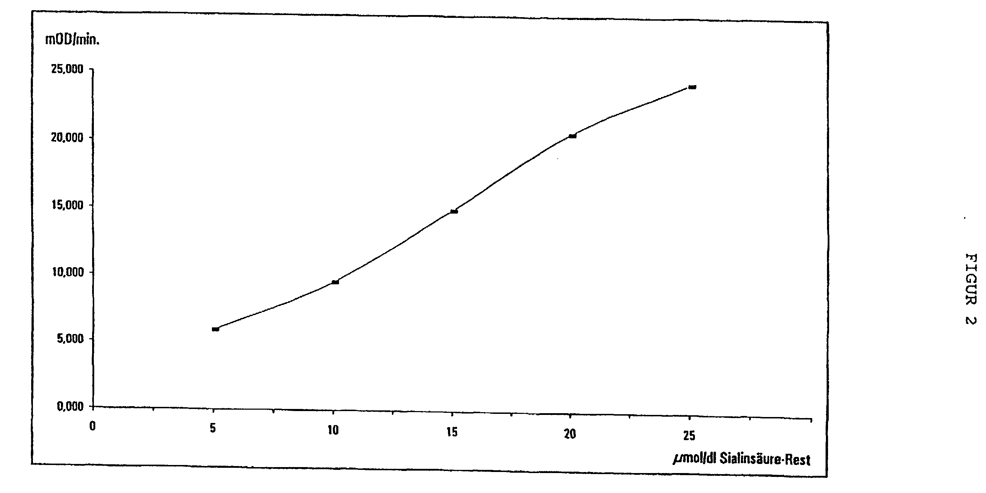

Figur 2 zeigt eine Standardkurve, die zur Auswertung einer erfindungsgemäßen SDT-Bestimmung geeignet sind. Wie gezeigt, werden in die Kurve die Werte von Standards mit 5, 10, 15, 20 und 25 $\mu$mol/dl SSR (Sialinsäurerest) eingetragen.

Die Vorteile der neuen Methode sind:

1. Hohe Spezifität, denn das Sambucus nigra-Lectin besitzt eine große Affinität für endständige $\alpha$-anomerische in 2-6-Stellung gebundene Sialinsäuren.

2. Große Sensibilität (von bis zu 5 ng im Test).

3. Sehr gute Reproduzierbarkeit.

4. Sehr hohe Präzision von Tag zu Tag und in der Serie.

5. Im Gegensatz zu den bisherigen Bestimmungsmethoden, die einen großen apparativen Aufwand und erfahrenes Personals erforderten, zeichnet sich dieses voll-immunoenzymatische Verfahren durch seine elegante direkte Bestimmungsmethode aus, die keine aufwendigen physikalisch-chemischen Trennverfahren zur Probenaufbereitung mehr benötigt und damit wesentlich einfacher, leichter verfügbar, sehr schnell durchzuführen und nicht anfällig für Arbeitsfehler in der Probenaufbereitungsphase ist.

6. Große Einfachheit des Testes, der problemlos als semi-quantitativer Test eingesetzt werden kann, der ohne Photometer mit bloßem Auge abgelesen werden kann.

7. Sehr kurze Reaktionszeit von weniger als 2 Stunden für 96 Bestimmungen pro Mikrotiterplatte.

Beispiele

Das in zwei Varianten vorgestellte EIA-Verfahren ist durch die Verwendung des spezifischen Sambucus nigra-Lectins gekennzeichnet, welches eine große Affinität zum $\alpha$-anomerischen endständigen Sialinsäure-Rest aufweist, welcher in 2-6-Stellung an die Galactose-N-acetyl-glucosamin-Kette gebunden ist.

Die Quantifizierung dieser Sialinsäure-Reste erfolgt in drei Etappen:

1. Bildung eines Antigen-Antikörper-Komplexes zwischen dem Transferrin und einem polyklonalen anti-Transferrin-Antikörper, der auf der Platte gebunden ist.

2. Bildung eines zusätzlichen molekularen spezifischen Komplexes zwischen Peroxidase-markiertem Sambucus nigra-Lectin und den Sialinsäure-Resten des Transferrins.

3. Oxidation eines Chromogens (ABTS) in Anwesenheit eines für Peroxidase spezifischen Substrats, dessen Farbintensität proportional zum Gehalt an der im Transferrin-Molekül enthaltenen Sialinsäure ist und bei 405 nm gemessen wird.

Beispiel 1

Quantitative Bestimmung der Sialinsäure-Reste humaner Isotransferrine (SDT = sialinsäuredefizientes Transferrin) durch eine immunolektinoenzymatische Reaktion unter Verwendung eines Sambucus nigra-Lectin-Konjugats.

a) Reaktionsprinzip

In der ersten Phase wird das Transferrrin von den restlichen Serumproteinen durch Anlagerung an einen humanen anti-Transferrin-Antikörper, der auf der Platten-Oberfläche fixiert wurde, abgetrennt.

In der zweiten Phase bilden die an den endständigen Oligosaccharid-Ketten des Transferrins gebundenen Sialinsäure-Reste einen stabilen Komplex mit dem Sambucus nigra-Lectin, welches mit Peroxidase markiert wurde.

Anschließend entwickelt sich durch die Einwirkung eines für Peroxidase spezifischen Substrats auf das Chromogen ABTS in Anwesenheit von Peroxidase eine grün-blaue Färbung, deren Intensität proportional zum Gehalt an Sialinsäure-Resten im Test ist.

b) Erforderliche Reagenzien

-   Verdünnungslösung für das Serum:

    PBS-Puffer: 0,01 M; pH 7,2- 7,4; 0,05% Tween[20] und 1% Albumin (BSA)

-   Wasch-Lösung:

    PBS-Puffer: 0,01 M; pH 7,2- 7,4;

-   Sambucus nigra-Lectin-Peroxidase Konjugat (kommerziell erhältlich von MEDAC Nr. H6801)
-   Citrat-Pufferlösung Substrat
    0,12 M Citratlösung, pH 4,0; 0,05% $H_2O_2$ (Peridrol)
-   ABTS-Substratlösung
    Citrat-Pufferlösung, enthaltend 1,5% ABTS (2,2'-Azino-bis-(3-ethyl)-benzthiazolin-6-sulfonsäure)
-   Stopp-Lösung
    Wäßrige 1 %ige Natriumdodecylsulfat-Lösung
-   Beschichtete Mikrotiterplatte mit anti-Transferrin-Antikörpern (Human anti-Transferrin-Antikörper aus Host animal, whole serum (Sigma Nr. T2027)

c) Vorbereitung der Mikrotiterplatten

c1) Vorbereitung der anti-Transferrin-Antikörper

-   Zu 4 ml anti-Transferrin-Antikörper Serum wird tropfenweise 2 ml gesättigte Ammoniumsulfatlösung [$(NH_4)_2SO_4$, MG 132,14, 900 g/l bidest. $H_2O$] zugegeben und mit einem Glasstäbchen vermischt.
-   60 min bei 4°C stehen lassen.
-   15 min bei 4°C zentrifugieren.
-   Überstand verwerfen und das Präzipitat in 2 ml PBS-Puffer (0,01 M; pH 7,2- 7,4) resuspendieren; diese letzten beiden Schritte 4 x wiederholen.
-   Das letzte Präzipitat in 1 ml PBS-Puffer resuspendieren und in Dialysevorrichtung einbringen (Dialysemembran: Fa. Serva, Heidelberg, Dialysis tubing Nr. 20/32, Best.Nr. 44110).
-   Dialyse bei 4°C gegen PBS-Pufferlösung, die nach jeweils zwei Stunden erneuert wird, bis diese auf Nessler-Reagenz (Nessler-Reagenz auf Ammoniumsalze, Merck-Nr. 1.09028) negativ reagiert.
-   Anschließend Bestimmung der Proteinkonzentration des gereinigten anti-Transferrin-Antikörpers nach Biuret (Soll-Konzentration ca. 20-50 mg/ml).

c2) Plattenbeschichtung

Die Polystyrol-Mikrotiterplatten werden mit 100 µl der hochgereinigten Lösung der polyklonalen anti-Transferrin-Antikörper (IgG) beschichtet. Zur Plattenbeschichtung soll die Endkonzentration der Lösung 20 mg Protein/ml in Carbonat-Pufferlösung (0,1 M, pH 9,6) betragen. Die Fixierung erfolgt bei 24-stündiger passiver Inkubation bei 4°C ohne Schüttler. Die Platten werden 3 mal mit der Waschlösung gewaschen und bei minus 20°C aufbewahrt.

d) Vorbereitung des Testserums:
Die Seren werden 1/10 mit der Verdünnungslösung verdünnt (100 µl + 900 µl).

e) Vorbereitung der Standards und Kontrollen

Die Serum-Standards mit 25, 20, 25, 10, 5, 2,5 µmol SSR/dl (SSR = Sialinsäurereste) und das Kontrollserum mit 20 µmol SSR/dl werden mit 0,5 ml bidestilliertem Wasser rekonstituiert.

f) Einsatz der Proben, Standards und Kontrollen

Eine Doppelbestimmung pro Test ist empfohlen. Je 100 µl der vorbereiteten Lösungen in die Plattenvertiefungen pipettieren. Für den Leerwert 100 µl der Verdünnungslösung in 2 Plattenvertiefungen pipettieren; 30 min bei Raumtemperatur bei gleichzeitigem Rütteln der Platte inkubieren.

Nach drei aufeinanderfolgenden Waschungen (immer bei den Leerwerten beginnen!) wird die feuchte Platte auf saugfähigem Papier vorsichtig abgeklopft.

g) Einsatz des Sambucus nigra-Lectin-Konjugats

Die Plattenvertiefungen werden mit 100 µl Konjugat-Lösung beschickt und 30 min bei Raumtemperatur und gleichzeitigem Rütteln der Platte inkubiert. Das spezifische Konjugat bildet mit den Sialinsäure-Resten, die parallel zum Transferrin/anti-Transferrin gebunden sind, einen stabilen Komplex. Nach drei aufeinanderfolgenden Waschungen wie zuvor wird die feuchte Platte auf saugfähigem Papier vorsichtig abgeklopft.

h) Einsatz des Substrats und des Chromogens

Die Plattenvertiefungen werden mit 100 µl ABTS-Substratlösung beschickt und 10 min bei Raumtemperatur und gleichzeitigem Rütteln der Platte inkubiert. Die Reaktion erfolgt mit einer grün-blauen Färbung des oxidierten Chromogens.

Die kinetische Messung dieser Reaktion erfolgt in 3 Punktmessungen im Zeitintervall von 5 min nach 10-minütiger Anfangsinkubation; die Reaktion kann durch Zugabe von 50 µl Stopp-Lösung (1% SDS) für eine Endpunktmessung nach 30 min gestoppt werden. Alternativ kann lediglich eine Endpunktmessung nach 30-minütiger Inkubation durchgeführt werden.

i) Messung

bei 405 nm mit einem Mikrotiterplatten-Photometer

j) Auswertung

Die Auswertung erfolgt mit einer zu erstellenden Konversionskurve:

1. Logarithmische Standard-Kurve

Die erhaltenen Extinktionsdifferenzen der Standards werden nach folgender Formel dezimal-logarithmisch gegen die dezimal-logarithmischen Standardkonzentrationen aufgetragen:

$$\text{Log. } \Delta E_{min} = \text{Log } \mu\text{mol/dl SSR}$$

2. Sigmoide Standard-Kurve

Die erhaltenen Extinktionsdifferenzen der Standards werden nach folgender Formel dezimal-logarithmisch gegen die Standardkonzentrationen aufgetragen:

$$\text{Log. } \Delta E = \frac{(a-d)}{[1 + (x/c)^b]} + d \ \mu\text{mol/dl SSR}$$

Das Kontrollserum mit 20 µmol SSR/dl sollte Extinktionsdifferenzen in folgenden Bereichen ergeben:

0,276 ± 10 % nach 15' (0,248 - 0,304)
0,426 ± 10 % nach 30' (0,383 - 0,492)
0,528 ± 10 % nach 60' (0,475 - 0,581)

Beispiel 2

Beispiel 1 wird wiederholt, außer daß anstelle des Sambucus nigra-Lectin-Peroxidasekonjugats biotinyliertes Sambucus nigra-Lectin (Fa. MEDAC, Nr. BA 6801) und ein Streptavidin-Peroxidasekonjugat verwendet wird.
Dazu werden in Schritt (g) 100 µl einer Lösung von biotinyliertem Lectin die Plattenvertiefungen pipettiert, 30 min bei Raumtemperatur und gleichzeitigem Rütteln der Platte inkubiert und nach dreimaligem Waschen je 100 µl Peroxidase-Streptavidin Konjugat Lösung zupipettiert, erneut 30 min bei Raumtemperatur unter Rütteln inkubiert und wie zuvor dreimal gewaschen.

Das Kontrollserum mit 20 µmol SSR/dl sollte Extinktionsdifferenzen in folgenden Bereichen ergeben:

0,353 ± 10 % nach 15' (0,318 - 0,388)
0,546 ± 10 % nach 30' (0,491 - 0,601)
0,572 ± 10 % nach 60' (0,515 - 0,629)

Beispiel 3

Herstellung eines Transferrin-freien Standards

Materialien

- Lectin Concavalin A (Con A) aus conavalia ensiformis (Jack bean) (Sigma Nr. C7275)
- N-Acetylneuramin-Lacto-N-neo-Tetraose c ($\alpha$ Neu 5 AC-(2-$\theta$-$\beta$Gal-(1-4)-$\beta$GlcNAc-(1-3)-$\beta$Gal-(1-4)-Glc, Natrium-salz, aus Humanmilch, MG: 1020,9 g/mol

   (Sigma Nr. A4814)

Nach Sättigung von Lectin Con A (spezifisch für $\alpha$-D-Glycosyl-Bindungen) mittels Sialinsäureresten in verschiedenen Konzentrationen (6-Punkt-Kalibration) wird eine Mikrotiterplatte mit dem gesättigten Lectin bzw. Komplex beschichtet. Bindung von Sambucus nigra-Lectin und Reaktion mit Substrat erfolgt wie in Beispiel 1, Schritt g) und h) beschrieben.

Alternativ wird zur Herstellung der Transferrin-freien Standards zunächst Lectin Con A im Überschuß auf der Mikrotiterplatte fixiert. Anschließend werden die Sialinsäurereste in den Konzentrationen 200, 100, 50, 25, 12,5 und 6,25 20 µmol SSR/l zu dem fixierten Lectin zupippettiert.

Beispiel 4      Vergleich

Für gesunde Männer und Frauen (nicht Schwangere) wird in der Literatur ein physiologischer Transferringehalt von 260 mg/dl (221-299 mg/dl) angegeben. Unter Zugrundelegung von Molekulargewichten von 77.000 g/Mol für Transferrin und von 308,3 g/Mol für SSR und durchschnittlich 5 SSR pro Transferrinmolekül errechnet sich daraus eine SSR-Konzentration im Bereich von etwa 14,35 µmol/dl bis 19,42 µmol/dl.

Als durchschnittlicher SSR-Serumgehalt für eine gesunde Kontrollgruppe wurde ein Wert von 50,51 µmol SSR/g Transferrin ermittelt (Stibler et al., Clinical and Experimental Research, Vol 10, No. 1, Jan/Feb 1986). Daraus ergeben sich unter Berücksichtigung einer relativen Abweichung vom Transferrin-Sollwert (ca. 260 mg/dl) von ±15% für den ermittelten Wert von 50,51 µmol SSR/g Transferrin folgende Bereiche:

|  | niedriger Bereich | hoher Bereich |
|---|---|---|
| µmol SSR/g Transferrin | 42,94 | 58,08 |
| µmol SSR/dl | 12,83 | 17,36 |
| erfindungsgemäß berechnet (s.o) gemessen | 14,35 | 19,42 |
|  | 14,18 | 19,33 |

Diese Ergebnisse zeigen, daß die Quantifizierung von Transferrin mit dem erfindungsgemäßen Verfahren zu Werten führt, die in guter Übereinstimmung zu berechneten und den in der Literatur veröffentlichten Werten stehen.

Die vorstehende Quantifizierung wurde durchgeführt gemäß dem Verfahren von Beispiel 1. Als Standard wurde Mucin mit deklariertem Gehalt von $\alpha$-anomerischen (2-6) Sialinsäureresten (Sigma No. M3895, Mucin von Bovine Submaxillary Glands) in Konzentrationen von 25, 20, 15, 10, 5 und 2,5 µmol SSR/dl verwendet.

**Patentansprüche**

1. Verfahren zur Bestimmung endständiger Sialinsäurereste von Human-Transferrin in einer Probenflüssigkeit nach einem Sandwich-Prinzip,
**dadurch gekennzeichnet,**
**dass** man die Probenflüssigkeit mit einem ersten, spezifisch mit Human-Transferrin bindefähigen Rezeptor inkubiert, den gebildeten Komplex von der Probenflüssigkeit abtrennt, mit einem zweiten, spezifisch mit endständigen Sialinsäureresten von Human-Transferrin bindefähigen Rezeptor, nämlich Sambucus nigra-Lectin, inkubiert, wobei der zweite Rezeptor an eine Markierung gebunden oder damit bindefähig ist, und den Komplex aus erstem Rezeptor, Human-Transferrin und zweitem Rezeptor über die Markierung bestimmt.

2. Verfahren nach Anspruch 1,
**dadurch gekennzeichnet,**
**dass** der erste Rezeptor ein anti-Transferrin-Antikörper ist.

3. Verfahren nach Anspruch 1,
**dadurch gekennzeichnet,**
**dass** der erste Rezeptor ein polyklonaler anti-Transferrin-Antikörper ist.

4. Verfahren nach einem der vorhergehenden Ansprüche,
**dadurch gekennzeichnet,**
**dass** der erste Rezeptor an eine Festphase gebunden ist oder mit einer Festphase bindefähig ist, wobei mindestens eine Inkubation oder/und Abtrennung des Komplexes in Gegenwart der Festphase durchgeführt wird.

5. Verfahren nach einem der vorhergehenden Ansprüche,
**dadurch gekennzeichnet,**
**dass** die Festphase die Wand eines Reaktionsgefäßes ist, ausgewählt aus Probenröhrchen, Mitrotiterplatte und Küvette, oder ein teilchenförmiges Material ist.

6. Verfahren nach einem der vorhergehenden Ansprüche,
**dadurch gekennzeichnet,**
**dass** der zweite Rezeptor biotinyliert ist und die Markierung an Streptavidin/Avidin gekoppelt ist.

7. Verfahren nach einem der vorhergehenden Ansprüche,
**dadurch gekennzeichnet,**
**dass** die Markierung eine Enzymmarkierung ist und die Bestimmung der Markierung die Bestimmung eines chromogenen, lumineszierenden oder fluoreszierenden Substrats des Enzyms oder einer Substrat-bedingten Lichtabsorptionsänderung umfasst.

8. Verfahren nach Anspruch 7,
**dadurch gekennzeichnet,**
**dass** das Enzym ausgewählt ist aus Peroxidase, Alkohol-Dehydrogenase, Glucose-6-Dehydrogenase oder Diaphorase.

9. Verfahren nach einem der Ansprüche 1 bis 6,
**dadurch gekennzeichnet,**
**dass** die Markierung eine Fluoreszenz- oder Lumineszenzmarkierung ist.

10. Verfahren nach einem der vorhergehenden Ansprüche,
**dadurch gekennzeichnet,**
**dass** die Probenflüssigkeit eine Human-Körperflüssigkeit ist.

11. Verfahren nach Anspruch 10,
**dadurch gekennzeichnet,**
**dass** die Human-Körperflüssigkeit ausgewählt wird aus Serum und Vollblut.

12. Verfahren zur Bestimmung von Sialinsäure-defizientem Human-Transferrin in Körperflüssigkeiten,
**dadurch gekennzeichnet,**

**dass** man

(a) in einer Probe einer Körperflüssigkeit eine Bestimmung nach einem Verfahren eines der vorhergehenden Ansprüche durchführt,

(b) eine Bestimmung von endständigen Sialinsäureresten an mindestens einem Standard durchführt, der eine Substanz, umfassend mit dem zweitem Rezeptor bindefähige Sialinsäurereste in definierter Menge enthält, und

(c) den Gehalt von Sialinsäure-defizientem Transferrin in einer Körperflüssigkeit auf Basis der Meßgrößen von Schritt (a) und Schritt (b) ermittelt..

**13.** Verfahren nach Anspruch 12,
**dadurch gekennzeichnet,**
**dass** man Schritt (b) mit drei bis sieben Standards, die jeweils eine unterschiedliche Menge von mit dem zweiten Rezeptor bindefähige Sialinsäureresten enthalten, durchführt und eine Eichkurve erstellt.

**14.** Verfahren nach Anspruch 12 oder 13,
**dadurch gekennzeichnet,**
**dass** der Standard Transferrin mit einer definierten Menge von Sialinsäureresten enthält.

**15.** Verfahren nach Anspruch 12 oder 13,
**dadurch gekennzeichnet,**
**dass** der Standard eine von Transferrin verschiedene Substanz mit einer definierten Menge von Sialinsäureresten enthält.

**16.** Verfahren nach Anspruch 15,
**dadurch gekennzeichnet,**
**dass** die Substanz ein festphasenseitiges Mucin oder/und ein festphasenseitiges Oligosaccharid mit definiertem Gehalt von mit dem zweitem Rezeptor bindefähigen Sialinsäureresten enthält.

**17.** Verfahren nach Anspruch 16,
**dadurch gekennzeichnet,**
**dass** die Substanz N-Acetylneuramin-Lacto-N-neo-Tetraose c ist, das an einer mit Concavalin A beschichteten Festphase bindefähig ist.

**18.** Verfahren zur Diagnose von Alkoholabusus,
**dadurch gekennzeichnet,**
**dass** man in einer Probe einer Körperflüssigkeit den Gehalt von Sialinsäure-defizientem Transferrin nach einem Verfahren eines der Ansprüche 14 bis 19 bestimmt und durch Vergleich mit Normalwerten eine pathologische Veränderung ermittelt.

**19.** Verwendung von Sambucus nigra-Lectin in einem Verfahren zur Bestimmung von Sialinsäure-defizientem Transferrin.

**20.** Verwendung eines Verfahrens nach einem der Ansprüche 12 bis 18 zur Diagnose von Alkoholabusus.

**21.** Verwendung eines Verfahrens nach einem der Ansprüche 12 bis 18 zur Therapieüberwachung bei Entzugspatienten.

**22.** Verwendung eines Verfahrens nach einem der Ansprüche 12 bis 18 bei der Differenzialdiagnose zur Unterscheidung von alkoholinduzierten von alkoholunabhängigen Erkrankungen.

**23.** Reagenzienkit zur Bestimmung endständiger Sialinsäurereste von Human-Transferrin, umfassend in räumlich getrennter Anordnung:

(a) festphasenseitigen, mit Human-Transferrin spezifisch bindefähigen ersten Rezeptor,
(b) zweiten, spezifisch mit endständigen Sialinsäureresten von Human-Transferrin bindefähigen Rezeptor, nämlich Sambucus nigra-Lectin, der an eine Markierung gebunden oder damit bindefähig ist und

gegebenenfalls übliche Hilfs-, Puffer- und Zusatzstoffe.

**24.** Reagenzienkit nach Anspruch 24,
**dadurch gekennzeichnet,**
**dass** der erste Rezeptor ein anti-Transferrin-Antikörper ist.

**25.** Reagenzienkit nach Anspruch 25,
**dadurch gekennzeichnet,**
**dass** der erste Rezeptor ein polyklonaler anti-Transferrin-Antikörper ist.

**26.** Reagenzienkit nach einem der Ansprüche 24 bis 26, weiterhin umfassend einen Standard, der eine Substanz, umfassend mit dem zweiten Rezeptor bindefähige Sialinsäurereste in definierter Menge enthält.

**27.** Teststreifen zur qualitativen oder semi-quantitativen Bestimmung von Sialinsäure-defizientem Transferrin, umfassend

(a) eine Probenauftragszone,
(b) mindestens eine Reaktionszone, enthaltend einen an einer Festphase immobilisierbaren ersten Rezeptor, der mit Human-Transferrin spezifisch bindefähig ist, und einen markierten zweiten Rezeptor, der mit endständigen Sialinsäureresten von Human-Transferrin spezifisch bindefähig ist nämlich Sambucus nigra-Lectin, und
(c) eine Nachweiszone zur Immobilisieerung von erstem Rezeptor, enthaltend eine mit dem ersten Rezeptor spezifisch bindefähige Substanz.

**28.** Teststreifen nach Anspruch 28,
**dadurch gekennzeichnet,**
**dass** der erste Rezeptor ein anti-Transferrin-Antikörper ist.

**29.** Teststreifen nach Anspruch 29,
**dadurch gekennzeichnet,**
**dass** der erste Rezeptor ein polyklonaler anti-Transferrin-Antikörper ist.

**30.** Teststreifen nach einem der Ansprüche 28 bis 30,
**dadurch gekennzeichnet,**
**dass** der erste Rezeptor biotinyliert ist und die Nachweiszonee Avidin oder Streptavidin umfasst.

**31.** Teststreifen nach einem der Ansprüche 28 bis 31,
**dadurch gekennzeichnet,**
**dass** die Markierung des zweiten Rezeptors ausgewählt ist aus Goldmarkierung, Silbermarkierung und Chromogenen.

**32.** Teststreifen nach einem der Ansprüche 28 bis 32, weiterhin umfassend mindestens eine Kontrollzone zur Negativ- oder/und Positivkontrolle.

**Claims**

**1.** Method for determining terminal sialic acid residues of human transferrin in a sample liquid based on the sandwich principle,
**characterized in that**
the sample liquid is incubated with a first receptor which is able to bind specifically to human transferrin, the complex that forms is separated from the sample liquid and incubated with a second receptor, namely Sambucus nigra lectin, which is able to bind specifically to terminal sialic acid residues of human transferrin the second receptor being bound to a label or having the ability to bind thereto, and the complex of first receptor, human transferrin and second receptor is determined by means of the label.

**2.** Method as claimed in claim 1,
**characterized in that**
the first receptor is an anti-transferrin antibody.

**3.** Method as claimed in claim 1,
**characterized in that**
the first receptor is a polyclonal anti-transferrin antibody.

**4.** Method as claimed in one of the previous claims,
**characterized in that**
the first receptor is bound to a solid phase or is able to bind to a solid phase and at least one incubation or/and separation of the complex is carried out in the presence of the solid phase.

**5.** Method as claimed in one of the previous claims,
**characterized in that**
the solid phase is the wall of a reaction vessel selected from sample tube, microtitre plate and cuvette or it is a particulate material.

**6.** Method as claimed in one of the previous claims,
**characterized in that**
the second receptor is biotinylated and the label is coupled to streptavidin/avidin.

**7.** Method as claimed in one of the previous claims,
**characterized in that**
the label is an enzyme label and the determination of the label comprises determining a chromogenic, luminescent or fluorescent substrate of the enzyme or of a substrate-induced change in light absorption.

**8.** Method as claimed in claim 7,
**characterized in that**
the enzyme is selected from peroxidase, alcohol dehydrogenase, glucose-6-dehydrogenase or diaphorase.

**9.** Method as claimed in one of the claims 1 to 6,
**characterized in that**
the label is a fluorescent or luminescent label.

**10.** Method as claimed in one of the previous claims,
**characterized in that**
the sample liquid is a human body fluid.

**11.** Method as claimed in claim 10,
**characterized in that**
the human body fluid is selected from serum and whole blood.

**12.** Method for the determination of sialic acid-deficient human transferrin in body fluids,
**characterized in that**

(a) a body fluid sample is assayed using a method as claimed in one of the previous claims,
(b) the terminal sialic acid residues of at least one standard are determined which contains a defined amount of a substance containing sialic acid residues capable of binding to the second receptor and
(c) the content of sialic acid-deficient transferrin in a body fluid is determined on the basis of the measured quantities in step (a) and step (b).

**13.** Method as claimed in claim 12,
**characterized in that**
step (b) is carried out with three to seven standards each of which contains a different amount of sialic acid residues capable of binding to the second receptor, and a calibration curve is plotted.

**14.** Method as claimed in claim 12 or 13,
**characterized in that**
the standard contains transferrin with a defined amount of sialic acid residues.

**15.** Method as claimed in claim 12 or 13,

**characterized in that**
the standard contains a substance other than transferrin with a defined amount of sialic acid residues.

16. Method as claimed in claim 15,
**characterized in that**
the substance contains an immobilized mucin or/and an immobilized oligosaccharide with a defined amount of sialic acid residues capable of binding to the second receptor.

17. Method as claimed in claim 16,
**characterized in that**
the substance is N-acetylneuramine-lacto-N-neotetraose c which is able to bind to a solid phase coated with concavalin A.

18. Method for diagnosing alcohol abuse,
**characterized in that**
the amount of sialic acid-deficient transferrin is determined in a sample of body fluid using a method as claimed in one of the claims 14 to 19 and then compared with normal levels to ascertain whether there is a pathological change.

19. Use of Sambucus nigra lectin in a method for determining sialic acid-deficient transferrin.

20. Use of a method as claimed in one of the claims 12 to 18 to diagnose alcohol abuse.

21. Use of a method as claimed in one of the claims 12 to 18 for monitoring therapeutic treatment of withdrawal patients.

22. Use of a method as claimed in one of the claims 12 to 18 for differential diagnosis to distinguish between alcohol-induced and alcohol independent diseases.

23. Reagent kit for determining terminal sialic acid residues of human transferrin containing in a spatially separate arrangement:

(a) a solid phase first receptor capable of binding specifically to human transferrin,
(b) a second receptor, namely Sambucus nigra lectin which is able to bind specifically to terminal sialic acid residues of human transferrin and is bound to or able to bind to a label and

optionally common auxiliary substances, buffers and additives.

24. Reagent kit as claimed in claim 23,
**characterized in that**
the first receptor is an anti-transferrin antibody.

25. Reagent kit as claimed in claim 24,
**characterized in that**
the first receptor is a polyclonal anti-transferrin antibody.

26. Reagent kit as claimed in one of the claims 23 to 25, additionally containing a standard which contains a defined amount of a substance containing sialic acid residues capable of binding to the second receptor.

27. Test strip for the qualitative or semi-quantitative determination of sialic acid-deficient transferrin comprising,

(a) a sample application zone,
(b) at least one reaction zone containing a first receptor which can be immobilized on a solid phase and which is able to bind specifically to human transferrin, and a labelled second receptor, namely Sambucus nigra lectin which is able to bind specifically with terminal sialic acid residues of human transferrin and
(c) a detection zone for immobilizing the first receptor which contains a substance which is capable to bind specifically to the first receptor.

28. Test strip as claimed in claim 27,

**characterized in that**
the first receptor is an anti-transferrin antibody.

29. Test strip as claimed in claim 28,
**characterized in that**
the first receptor is a polyclonal anti-transferrin antibody.

30. Test strip as claimed in one of the claims 27 to 29,
**characterized in that**
the first receptor is biotinylated and the detection zone contains avidin or streptavidin.

31. Test strip as claimed in one of the claims 27 to 30,
**characterized in that**
the label of the second receptor is selected from gold label, silver label and chromogens.

32. Test as claimed in one of the claims 27 to 31,
additionally comprising at least one control zone for a negative or/and positive control.

**Revendications**

1. Procédé pour la détermination de groupes terminaux acide sialique de la transferrine humaine dans un liquide d'essai ou d'échantillon selon un principe en sandwich,
**caractérisé en ce que**,
l'on fait incuber le liquide d'essai avec un premier récepteur, spécifiquement capable de se fixer sur la transferrine humaine, on sépare le complexe formé du liquide d'essai, on fait incuber avec un second récepteur spécifiquement capable de se fixer sur les groupes terminaux acide sialique de la transferrine humaine, en l'occurrence Sambucus nigra-lectine, moyennant quoi le second récepteur est fixé sur un marquage ou est capable de se fixer sur celui-ci et on détermine le complexe provenant du premier récepteur, transferrine humaine et le deuxième récepteur par le biais du marquage.

2. Procédé selon la revendication 1,
**caractérisé en ce que**,
le premier récepteur est un anticorps anti-transferrine.

3. Procédé selon la revendication 1,
**caractérisé en ce que**,
le premier récepteur est un anticorps anti-transferrine polyclonal.

4. Procédé selon l'une des revendications précédentes,
**caractérisé en ce que**,
le premier récepteur est fixé sur une phase solide ou peut être fixé sur celle-ci, moyennant quoi on effectue au moins une incubation ou/et une séparation du complexe en présence de la phase solide.

5. Procédé selon l'une des revendications précédentes,
**caractérisé en ce que**,
la phase solide est la paroi d'un réacteur, sélectionné à partir d'un tube d'essai, plaque de microtitrage et cuvette ou un matériau particulaire.

6. Procédé selon l'une des revendications précédentes,
**caractérisé en ce que**,
le second récepteur est biotinylé et le marquage est couplé à la streptavidine/avidine.

7. Procédé selon l'une des revendications précédentes,
**caractérisé en ce que**,
le marquage est un marquage enzymatique et la détermination du marquage comprend la détermination d'un substrat chromogène, luminescent ou fluorescent de l'enzyme ou une modification de l'absorption de la lumière conditionnée par le substrat.

**8.** Procédé selon la revendication 7,
**caractérisé en ce que**,
l'enzyme est sélectionnée à partir de la peroxydase, de l'alcool-déshydrogénase, glucose-6-déshydrogénase ou diaphorase.

**9.** Procédé selon l'une des revendications 1 à 6,
**caractérisé en ce que**,
le marquage est un marquage de fluorescence ou de luminescence.

**10.** Procédé selon l'une des revendications précédentes,
**caractérisé en ce que**,
le liquide d'essai est un liquide corporel humain.

**11.** Procédé selon la revendication 10,
**caractérisé en ce que**,
le liquide corporel humain est sélectionné à partir de sérum et de sang complet.

**12.** Procédé pour la détermination de transferrine humaine déficiente en acide sialique dans les liquides corporels,
**caractérisé en ce que**,

(a) dans un échantillon d'un liquide corporel, on effectue une détermination de l'une des revendications précédentes,

(b) on effectue une détermination des groupes terminaux acide sialique sur au moins un standard qui contient une substance incorporant des groupes acide sialique pouvant se fixer sur le second récepteur en quantité définie, et

(c) on calcule la teneur en transferrine déficiente en acide sialique dans un liquide corporel sur la base des grandeurs de mesure de l'étape (a) et de l'étape (b).

**13.** Procédé selon la revendication 12,
**caractérisé en ce que**,
l'on effectue l'étape (b) avec trois jusqu'à sept standards qui contiennent respectivement une quantité différentielle de groupes acide sialique pouvant se fixer sur le second récepteur et on établit une courbe d'étalonnage.

**14.** Procédé selon la revendication 12 ou 13,
**caractérisé en ce que**,
le standard contient de la transferrine avec une quantité définie de groupes acide sialique.

**15.** Procédé selon la revendication 12 ou 13,
**caractérisé en ce que**,
le standard contient une substance différente de la transferrine avec une quantité définie de groupes acide sialique.

**16.** Procédé selon la revendication 15,
**caractérisé en ce que**,
la substance contient une mucine côté phase solide ou/et un oligosaccharide côté phase solide avec une teneur définie de groupes acide sialique pouvant se fixer sur le second récepteur.

**17.** Procédé selon la revendication 16,
**caractérisé en ce que**,
la substance est le N-acétylneuramine-lacto-n-néo-tétraose c qui peut se fixer sur une phase solide revêtue de concavaline A.

**18.** Procédé pour le diagnostic de l'abus alcoolique,
**caractérisé en ce que**,
l'on détermine dans un essai ou échantillon d'un liquide corporel la teneur en transferrine déficiente en acide sialique selon un procédé de l'une des revendications 14 à 19 et l'on établit une modification pathologique par comparaison avec des valeurs normales.

**19.** Utilisation de Sambucus nigra-lectine dans un procédé pour la détermination de transferrine déficiente en acide sialique.

**20.** Utilisation d'un procédé selon l'une des revendications 12 à 18 pour le diagnostic de l'utilisation abusive de boissons alcooliques.

**21.** Utilisation d'un procédé selon l'une des revendications 12 à 18 destiné à la surveillance thérapeutique chez des patients en cours de désintoxication.

**22.** Utilisation d'un procédé selon l'une des revendications 12 à 18 dans le diagnostic différentiel pour distinguer les pathologies induites par l'alcool de celles indépendantes de l'alcool.

**23.** Trousse ou kit de réactifs pour la détermination de groupes terminaux acide sialique de la transferrine humaine, comprenant en disposition spatialement séparée :

(a) un premier récepteur côté phase solide, pouvant spécifiquement se fixer sur la transferrine humaine,

(b) un second récepteur, pouvant se fixer spécifiquement sur les groupes terminaux acide sialique de la transferrine humaine, en l'occurrence Sambucus nigra-lectine, qui est fixé sur un marquage ou peut se fixer sur celui-ci et

éventuellement, les adjuvants, additifs et produits tampons usuels.

**24.** Trousse ou kit de réactifs selon la revendication 24,
**caractérisé en ce que**,
le premier récepteur est un anti-corps anti-transferrine.

**25.** Trousse ou kit de réactifs selon la revendication 25,
**caractérisé en ce que**,
le premier récepteur est un anti-corps anti-transferrine polyclonal.

**26.** Trousse ou kit de réactifs selon l'une des revendications 24 à 26, comprenant de plus un standard qui contient une substance, incorporant des groupements acide sialique en quantité définie pouvant se fixer sur le second récepteur.

**27.** Bande d'essai pour la détermination qualitative ou semi-quantitative de transferrine déficiente en acide sialique, comprenant :

(a) une zone d'application de l'échantillon,

(b) au moins une zone de réaction, contenant un premier récepteur pouvant être immobilisé sur une phase solide, qui peut spécifiquement se fixer sur la transferrine humaine, et un second récepteur marqué, qui peut spécifiquement se fixer sur la transferrine humaine, en l'occurrence Sambucus nigra-lectine, et

(c) une zone de mise en évidence pour l'immobilisation du premier récepteur, contenant une substance pouvant se fixez spécifiquement sur le premier récepteur.

**28.** Bande de test selon la revendication 27,
**caractérisée en ce que**,
le premier récepteur est un anti-corps anti-transferrine.

**29.** Bande de test selon la revendication 28,
**caractérisée en ce que**,
le premier récepteur est un anti-corps anti-transferrine polyclonal.

**30.** Bande de test selon l'une des revendications 27 à 29,
**caractérisée en ce que**,
le premier récepteur est biotinylé et la zone de mise en évidence contient de l'avidine ou de la streptavidine.

**31.** Bande de test selon l'une des revendications 27 à 30,
**caractérisée en ce que**,
le marquage du second récepteur est choisi à partir d'un marquage or, d'un marquage argent et chromogène.

**32.** Bande de test selon l'une des revendications 27 à 31, comprenant de plus au moins une zone de contrôle pour le contrôle négatif ou/et positif.

# Tetrasialotransferrin

| Sialinsäure | Sialinsäure | Sialinsäure | Sialinsäure |
| --- | --- | --- | --- |
| $\mid$ 2-6 | $\mid$ 2-6 | $\mid$ 2-6 | $\mid$ 2-6 |
| Galactose | Galactose | Galactose | Galactose |
| $\mid$ 1-3 (4) | $\mid$ 1-3 (4) | $\mid$ 1-3 (4) | $\mid$ 1-3 (4) |
| N-acetyl-glucosamin | N-acetyl-glucosamin | N-acetyl-glucosamin | N-acetyl-glucosamin |
| $\mid$ 1-3 | $\mid$ 1-3 | $\mid$ 1-3 | $\mid$ 1-3 |

Mannose          Mannose                    Mannose          Mannose

Mannose                                 Mannose

N-acetyl-glucosamin                     N-acetyl-glucosamin

N-acetyl-glucosamin                     N-acetyl-glucosamin

Polypeptidkette - - - - - - - - - - - - - - - - - - - - - - - - - - - - - - - - - - - - - - - - - - - - - - - - - - - - - - - - - - - - - - -

FIGUR 1

FIGUR 2